# EUROPEAN PATENT SPECIFICATION

(11) **EP 2 078 567 B1**
(45) Date of publication and mention of the grant of the patent: **09.06.2010**
(21) Application number: 07830645.3
(22) Date of filing: 26.10.2007
(51) Int. Cl.: B05B 17/06, D06F 39/08

(54) **ELECTROLYTIC MIST GENERATOR AND WASHING MACHINE USING IT**
ELEKTROLYTISCHER NEBELERZEUGER UND DIESEN VERWENDENDE WASCHMASCHINE
GENERATEUR DE BROUILLARD ELECTROLYTIQUE ET MACHINE A LAVER L'UTILISANT

(30) Priority: 20.11.2006 JP 2006312604
(43) Date of publication of application: 15.07.2009
(73) Proprietor: Panasonic Corporation, Kadoma-shi Osaka 571-8501 (JP)
(72) Inventor: FUJII, Hiroyuki, c/o PANASONIC CORPORATION, Shiromi 2-chome Chuo-ku, Osaka-shi, Osaka 540-6207 (JP); NUMOTO, Hironao, c/o PANASONIC CORPORATION, Shiromi 2-chome Chuo-ku, Osaka-shi, Osaka 540-6207 (JP); ASAMI, Tadashi, c/o PANASONIC CORPORATION, Shiromi, 2-chome Chuo-ku, Osaka-shi, Osaka 540-6207 (JP)
(74) Representative: Schwabe - Sandmair - Marx
(86) International application number: PCT/JP2007/070911
(87) International publication number: WO 2008/062635

(56) References cited:
- JP-A- 60 082 164
- JP-A- 2002 095 728
- JP-A- 2006 102 528

## Description

### TECHNICAL FIELD

The present invention relates to an electrolytic mist generating device for disinfecting and antibacterial effects, and a washing machine using the same.

### BACKGROUND ART

In recent years, technology for subjecting laundry to antibacterial treatment during washing has been proposed. For example, Patent Literature 1 discloses an electric washing machine with an ion generator that generates a metal ion having bacteriocidal capacity. Patent Literature 2 discloses a washing machine with an Ag ion adding unit that adds an Ag ion to cleaning water. Patent Document 3 discloses a washing machine that water with an Ag ion eluted is sprayed onto clothing in a shower-like manner. Patent Literature 4 discloses a drier that squirts, on laundry, mist of water with an Ag ion eluted.

Topically, the elution of a metal ion is performed by electrolysis. Applying a voltage between electrodes for electrolysis allows the metal ion to be eluted from the anodic electrode in accordance with Faraday's law.

However, in Patent Literatures 1 and 2, the water with the metal ion eluted is used in rinsing, and thus, the metal ion not attached to the clothing is discarded as drainage. Most of the metal ion thus goes to waste. Moreover, in Patent Literatures 3 and 4, the elution of the Ag ion is performed by a flow method using a flow path from water service, and thus, only electrolytic water of low-concentrated Ag ion is created. Therefore, a great amount of electrolytic water is needed for obtaining the Ag ion that brings about the antibacterial effect to the washed clothing. In this case, since a piezoelectric element cannot generate so much mist per unit time, processing time becomes very long.
[Patent Literature 1] Unexamined Japanese Utility Model Publication No. H05-74487
[Patent Literature 2] Unexamined Japanese Patent Publication No. 2001-276484
[Patent Literature 3] Unexamined Japanese Patent Publication No. 2005-87712
[Patent Literature 4] Unexamined Japanese Patent Publication No. 2006-141579

### DISCLOSURE OF THE INVENTION

One aspect of the present invention is to provide an electrolytic mist generating device including an electrolytic cell, electrode parts where a pair of positive pole and negative pole are arranged in parallel and opposed to one another in the electrolytic cell, a piezoelectric element that generates mist of electrolytic water generated inside the electrolytic cell, a water supply device that supplies water into the electrolytic cell, a discharging device that discharges the mist from the electrolytic cell, and a control circuit that controls operation steps of the electrolytic cell, wherein in an electrolysis step, a voltage that does not permit the mist to be generated is applied to the piezoelectric element to generate a water column. Since in the electrolysis step, by applying to the piezoelectric element the voltage that does not permit the mist to be generated to generate the water column, the electrolysis in the electrode parts is performed by a batch method, desired high-concentration metal ion water can be stably and continuously obtained in accordance with an electrolytic current value and an electrolysis time. By applying to the piezoelectric element the voltage that does not permit the mist to be generated to generate the water column, aqueous solution during the electrolysis step can be efficiently circulated, so that a harmful effect such as nonuniformity of the solution accompanying the batch method and the like can be prevented. The piezoelectric element can have both the mist generation function and the water circulating function in the electrolytic cell.

Preferably, the control circuit executes a drainage step of discharging electrolytic water in the electrolytic cell. With this configuration, discharging the aqueous solution with a metal ion eluted by electrolysis after the mist supply can prevent the aqueous solution remaining until next use from changing in quality, and settling out and accumulating to do harm to the device.

Preferably, the control circuit executes a water supply step of supplying water into the electrolytic cell after the drainage step. With this configuration, since a series of steps associated with the electrolytic mist generation end in a state where the water is supplied, all the times, the inside of the electrolytic cell is not dry, and can be maintained in a state where solid materials hardly occur.

Preferably, the control circuit executes a cleaning step of cleaning an inside of the electrolytic cell after the drainage step. By adding the cleaning step, the electrode parts and the inside of the electrolytic cell can be cleaned, and deposits and the like inside the electrolytic cell can be restrained for a long time, so that a stable state for the electrolysis can be maintained.

Preferably, the control circuit executes a water supply step of supplying water into the electrolytic cell after the cleaning step. With this configuration, since the series of steps associated with the electrolytic mist generation end in the state where the water is supplied, all the times, the inside of the electrolytic cell is not dry, and can be maintained in the state where solid materials hardly occur.

Preferably, the voltage that does not permit the mist to be generated and is applied to the piezoelectric element may be 1/3 or more and 1/2 or less of a rated voltage that permits the mist to be generated. With this configuration, although the water column generated from the piezoelectric element extends above a water surface, it falls in a state where the mist generation by water crushing is not reached. As a result, a circulating water path from the piezoelectric element toward a water column falling direction can be formed, so that the electrolytic state can be made uniform from the electrode parts to the whole aqueous solution inside the electrolytic cell.

Preferably, the water column generated from the piezoelectric element may be formed by a water flow passing though the electrode parts. With this configuration, the water circulation in the electrolysis step can be smoothly performed, and harmful effects such as nonuniformity of the solution accompanying the batch method can be prevented.

Preferably, the electrolytic cell may have a recessed part having a predetermined depth in a bottom portion thereof, and the piezoelectric element may be arranged in the recessed part so as to be inclined. With this configuration, a volume of stored water at a level required for protecting the piezoelectric element can be made as small as possible, and a volume of the aqueous solution with the metal ion eluted that is involuntarily wasted as drainage after the mist processing step can be reduced.

Preferably, the electrolysis step may be controlled by a constant current circuit. With this configuration, even if properties of the service water change to some extent, the electrolytic aqueous solution containing a certain amount of Ag can be obtained, and uniform effects to the clothing can be brought about.

Another aspect of the present invention is to provide a washing machine including the above-described electrolytic mist generating device, a washing tub that contains laundry, an outer tub in which the washing tub is internally mounted rotatably, and a water supply device that supplies washing water to the outer tub, wherein mist of electrolytic water generated by the electrolytic mist generating device is supplied to the washing tub. This can bring about disinfecting and antibacterial actions to the clothing, and a mildewproofing action to the washing tub as well. By setting an attachment position of the electrolytic mist generating device to a front-surface-side upper portion of horizontal or oblique type washing tub, a user can also observe contact of the generated mist having an average particle diameter of 10 µm with the washed clothing inside the washing tub, so that the user can visually check the clothing processing step with the mist to obtain a visual effect of the mist processing.

Preferably, an inner surface of an introduction part from the electrolytic mist generating device to the washing tub may be subjected to water repellent treatment. This allows the generated mist to reach the washed clothing while restraining, as much as possible, loss of the mist due to attachment to a wall surface until it is introduced to the washing tub.

Preferably, the electrolytic mist generating device may supply mist of Ag ion electrolytic water of 20 ppm or more and 200 ppm or less to the washing machine. With this configuration, the high-concentration electrolytic ion aqueous solution can reduce a liquid volume required for the clothing, resulting in a reduction in mist processing time.

Preferably, the supply of the mist to the washing tub may be performed while laundry is tumbled after a final spin drying step. With this configuration, since after the high-concentration electrolytic ion aqueous solution is attached to the washed clothing, it further wets and spreads by utilizing the water contained in the clothing, unevenness in the mist attachment could be corrected.

### BRIEF DESCRIPTION OF THE DRAWINGS

Fig. 1 is a configuration view of an electrolytic mist generating device of Embodiment 1.
Fig. 2 is a cross-sectional view of Fig. 1.
Fig. 3 is a top view showing an arrangement of electrode parts and a piezoelectric element inside an electrolytic cell.
Fig. 4 is a cross-sectional view of a washing and drying machine mounting the electrolytic mist generating device.
Fig. 5 is a back view of Fig. 4.
Fig. 6 is a system flowchart of the electrolytic mist generating device of Embodiment 1.
Fig. 7 is a configuration view of an electrolytic mist generating device of Embodiment 2.
Fig. 8 is a system flowchart of an electrolytic mist generating device of Embodiment 3.
Fig. 9 is a system flowchart of an electrolytic mist generating device of Embodiment 4.

### DESCRIPTION OF REFERENCE MARKS

- 1: electrolytic cell
- 2: electrode part
- 4: piezoelectric element
- 5: recessed part
- 6: rectifier
- 7: blowing fan
- 8: mist outlet
- 9: water supply and drain port
- 10: body
- 12: cylindrical outer tub
- 13: clothing
- 14: cylindrical inner tub
- 15: drive motor
- 40: electrolytic mist generating device
- 41: mist introducing path
- 50: water supply device
- 70: discharging device
- 80: control circuit

### PREFERRED EMBODIMENT FOR CARRYING OUT OF THE INVENTION

Hereinafter, embodiments of the present invention are described with reference to the drawings. These embodiments, however, do not limit the present invention.

### (Embodiment 1)

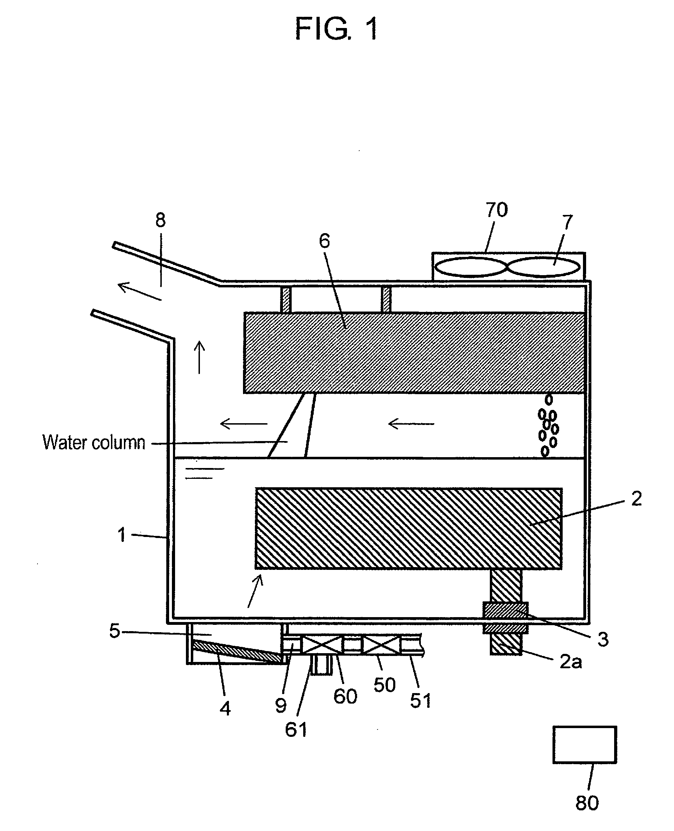

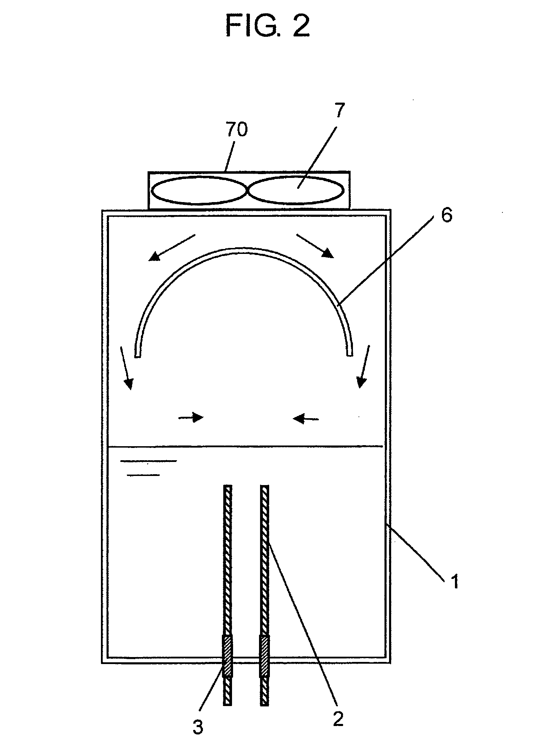

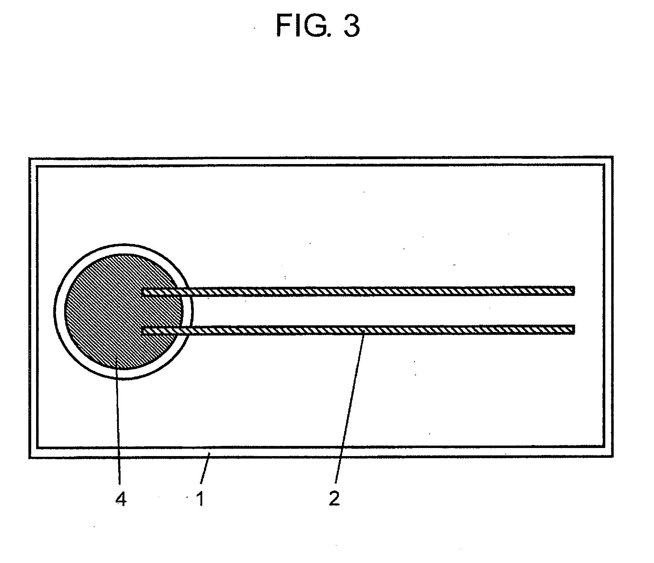

Fig. 1 is a schematic configuration view of an electrolytic mist generating device according to Embodiment 1 of the present invention, Fig. 2 is a cross-sectional view of Fig. 1, and Fig. 3 is a top view of electrode parts and a piezoelectric element.

Two sheets of electrode parts 2 are arranged in parallel with respect to a bottom surface of electrolytic cell 1. Electrode parts 2 are specifically Ag plates of 2 cm x 5 cm having a thickness of 1.2 mm, and spaced from each other at a distance of 8 mm. Terminal part 2a for applying a voltage through rubber packing 3 from each of electrode parts 2 is drawn outside electrolytic cell 1. Each of electrode parts 2 is fixed to the bottom surface of electrolytic cell 1 by rubber packing 3.

Piezoelectric element 4 is arranged adjacent to a position where a generated water column is formed by a water flow passing between two sheets of electrode parts 2. In a bottom portion, there is provided recessed part 5 having a predetermined depth of 15 mm from the bottom surface of electrolytic cell 1, where piezoelectric element 4 is disposed so as to keep an inclination of about 10 degrees. As a result, the water column generated from piezoelectric element 4 is formed with an angle in an oblique direction and is formed by the water flow passing between electrode parts 2 spaced from each other at a distance of 8 mm, so that a circulating water path from piezoelectric element 4 toward a water column falling direction can be formed. Specifically, piezoelectric element 4 of φ20 mm and 1.6 MHz for rated AC 48V is used.

Moreover, above electrode parts 2, rectifier 6 having a substantially semi-cylindrical shape is disposed in a portion at a certain distance from a top surface position of electrolytic cell 1 so as to be fixed to a top surface portion. Specifically, it has a semi-cylindrical shape of φ35 mm x 60 mm and is made of glass having thickness of 1 mm. Rectifier 6 is laid out so that during the mist supply, the water column generated from piezoelectric element 4 crashes against rectifier 6 having the substantially semi-cylindrical shape while performing water crushing, and that the water then falls between two sheets of electrode parts 2 to be returned.

On the top surface of electrolytic cell 1 about 10 mm above rectifier 6 having the substantially semi-cylindrical shape, there is disposed blowing fan 7 as discharging device 70 that discharges mist generated inside electrolytic cell 1. Blowing fan 7 is made of an axial flow fan having a diameter of 30 mm. A configuration is made such that wind from blowing fan 7 is blocked off by rectifier 6 having the substantially semi-cylindrical shape, passes through clearances of about 5 mm between an inner wall surface of electrolytic cell 1 and rectifier 6, and flows in an inside direction, so that the wind is supplied to the water column generated from the piezoelectric element 4 from a side surface direction. In a side-surface upper portion of electrolytic cell 1, there is disposed mist outlet 8.

Moreover, in recessed part 5 of electrolytic cell 1 where piezoelectric element 4 is disposed, water supply and drain port 9 is provided. Switching valve 60 connects water supply and drain port 9 to water supply device 50 during water supply and to drain pipe 61 during drainage. Water service pipe 51 is connected to water supply device 50. Control circuit 80 arranged in the vicinity of electrolytic cell 1 controls operation steps of electrolytic cell 1 through coupling not shown. A water level sensor (not shown) is provided in electrolytic cell 1 to perform water-supply upper-limit level management to electrolytic cell 1, and lower-limit level management when the mist is generated.

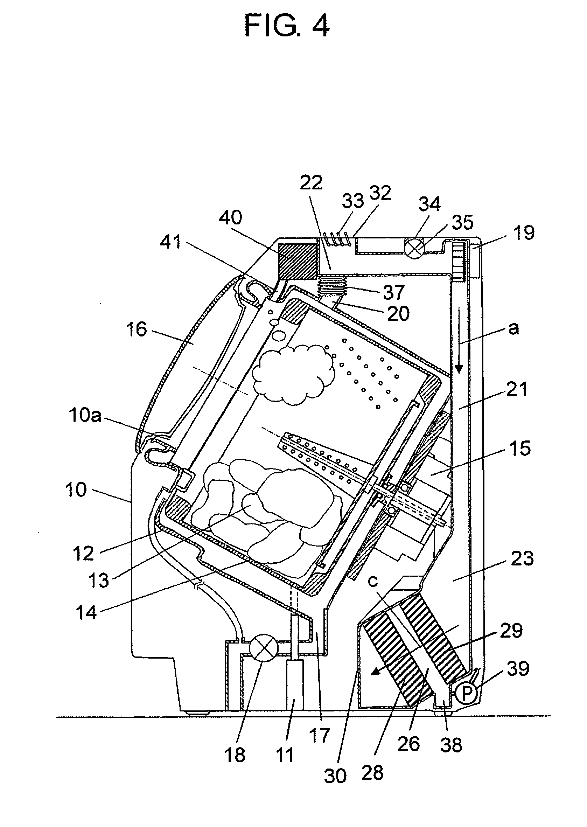

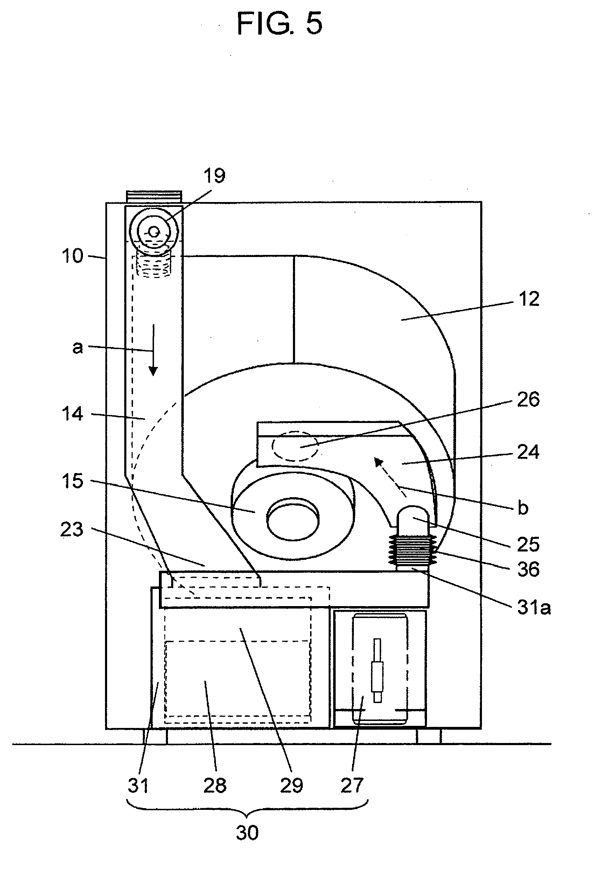

Fig. 4 is a cross-sectional view of a washing and drying machine with the electrolytic mist generating device of Embodiment 1 of the present invention installed, and Fig. 5 is a back view of Fig. 4.

Cylindrical outer tub 12 supported elastically by a plurality of suspensions 11 is provided inside body 10, and vibrations during washing and spin drying are absorbed by suspensions 11. Inside outer tub 12, cylindrical inner tub 14 to contain clothing 13 is rotatably provided, and is rotatively driven by drive motor 15 as a drive device. Outer tub 12 serves as a washing chamber of clothing 13 in a washing step and serves as a drying chamber of clothing 13 in a drying step.

Opening part 10a for taking clothing 13 in and out, and door 16 for opening and closing the same are provided in a front surface of body 10. Door 16 is made of transparent glass so that the clothing inside the washing tub can be observed. Outer tub 12 and inner tub 14 also have similar opening parts on the front surface side thereof, and this opening part of outer tub 12 is watertightly joined to opening part 10a of body 10 by a bellows. In a bottom portion of outer tub 12, drain port 17 for discharging washing water is provided, and is joined to drain valve 18 that opens and closes a drain path. Drain valve 18 is closed during washing so that a predetermined volume of washing water can be stored in outer tub 12. Blower 19 as a blowing device is provided in an upper portion of body 10.

Blower 19 suctions drying air, which has passed through inner tub 14 and outer tub 12, from outer tub outlet 20 provided above outer tub 12, and blow an inside of upstream circulating air path 21 provided in a back surface of outer tub 12 to derive the air from upstream circulating air-path inlet 22 to upstream circulating air-path outlet 23 as indicated by arrow a. Moreover, downstream circulating air path 24 is provided in an outer surface of outer tub 12, and the drying air coming in through downstream circulating air-path inlet 25 is blown in a direction of arrow b to be supplied into outer tub 12 and inner tub 14 through blowing port 26.

In a back-surface lower portion of outer tub 12 is arranged heat pump device 30 in which compressor 27, radiator 28 that radiates heat of a compressed refrigerant, a pressure reducing device (not shown) for reducing a pressure of the high-pressure refrigerant, and heat absorber 29 in which the low-pressure refrigerant resulting from the reduction of pressure removes heat from a vicinity are joined to one another by pipe line so as to circulate the refrigerant through them, and an empty space inside body 10 is effectively utilized to house heat pump device 30. Heat-exchanging air path 31 serves to cause the air blown by blower 19 to flow from heat absorber 29 to heat radiator 28 in a direction of arrow c, and contains compressor 27 alongside of heat absorber 29 and heat radiator 28 in a lateral direction of body 10 inside heat-exchanging air path 31. An inlet side of heat-exchanging air path 31 is communicated with upstream circulating air-path outlet 23, and an outlet side thereof is communicated with downstream circulating air-path inlet 25.

In upstream circulating air path 21 from outer tub 12 to heat absorber 29, exhaust port 32 for exhausting the air flowing here outside body 10 is provided in an upper surface of body 10. Freely opened and closed louver 33 is provided in exhaust port 32 so as to enable selection as to whether or not to perform the exhaust from exhaust port 32 and adjustment of an exhaust direction.

Moreover, intake port 34 for taking in external air is provided downstream of exhaust port 32 in upstream circulating air path 21. Intake port 34 is located between exhaust port 32 and blower 19, and an opening and closing device of intake port 34 is made of intake valve 35 consisting of an opening and closing valve such as an electromagnetic valve so as to enable selection as to whether or not to perform the intake.

Downstream circulating air-path inlet 25 and heat-exchanging air-path outlet 31a are communicated through air supply hose 36 made of a flexible material, which is capable of extension and contraction in a bellows-like manner, and outer tub outlet 20 and upstream circulating air-path inlet 22 are communicated through exhaust hose 37 made of a flexible material, which is capable of extension and contraction in a bellows-like manner, which prevents the vibrations of outer tub 12 from being transmitted to heat pump device 30. Moreover, in a lower portion of heat-exchanging air path 31, drain water vessel 38 for collecting dehumidified water from heat absorber 29 is provided, and the water collected in drain water vessel 38 is discharged outside the machine from drain pump 39.

In heat pump device 30, compressor 27, radiator 28 that radiates heat of the compressed refrigerant, the pressure reducing device for reducing the pressure of the high-pressure refrigerant and made of a throttle valve, a capillary and the like, and heat absorber 29 in which the low-pressure refrigerant resulting from the reduction of pressure removes heat from the vicinity are joined to one another by pipe line so as to circulate the refrigerant through them, so that a heat pump cycle is realized.

Electrolytic mist generating device 40 is arranged in a front upper portion of body 10. Mist introducing path 41 joins electrolytic mist generating device 40 and outer tub 12 to lead generated mist to inner tub 14, thereby enabling mist processing for clothing 13. Mist introducing path 41 is subjected to water repellent treatment in order to restrain mist attachment to a path wall surface as much as possible. Specifically, fluorine resin coating is applied to the same.

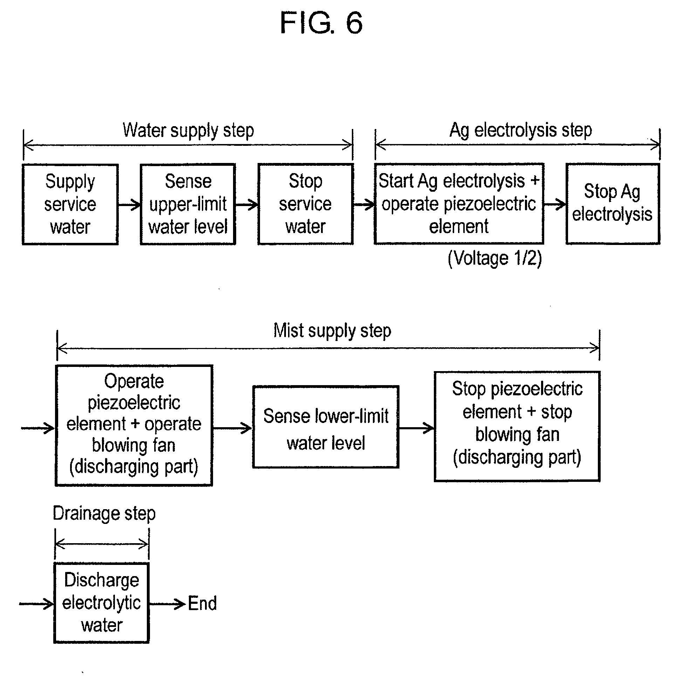

Next, operation of the electrolytic mist generating device is described. Fig. 6 is a system flowchart showing the operation of the electrolytic mist generating device.

First, a water supply step of supplying service water into electrolytic cell 1 up to a predetermined water level is performed. Water supply device 50 supplies water into electrolytic cell 1 through switching valve 60 and water supply and drain port 9 and stops the water supply when the predetermined water level is sensed by the water level sensor. For example, service water of about 100 ml having a hardness of about 40 and an electric conductivity of 150 µS/cm is supplied, so that the water level in electrolytic cell 1 becomes 30 mm from the bottom surface.

Thereafter, as an electrolysis step, AC 24 V is applied to piezoelectric element 4 as a voltage that does not permit the mist to be generated, thereby enabling the water inside electrolytic cell 1 to circulate therethrough. Almost concurrently, a voltage is applied utilizing a constant current circuit so as to pass DC 30 mA through electrode parts 2. The water column generated from piezoelectric element 4 is formed by a water flow passing between electrode parts 2, and returning water after forming the water column in an oblique upper direction also falls between electrode parts 2, thereby increasing an effect of putting the electrolytic water into a uniform state. Electrolysis is performed for a total of 200 seconds while a polarity of the electrodes to which the voltage is applied is inverted every about 20 seconds. At this time, about 15 V is applied as a DC voltage. As a result, an aqueous solution of about 50 ppm containing an Ag ion and AgCl, which is cloudy to some extent, is obtained.

Thereafter, as a mist supply step, AC 48 V is applied to piezoelectric element 4 to produce a state capable of generating the mist. At the same time, blowing fan 7 and drive motor 15 are also activated. Although the water column generated from piezoelectric element 4 crashes against rectifier 6 while performing water crushing, it does not lose retained energy after crashing against rectifier 6, and then, water detachment is created by blowing fan 7. As a result, the mist generated by piezoelectric element 4 is discharged from mist outlet 8 by blowing fan 7 and arrives at washed clothing 13 in inner tub 14 through mist introducing path 41.

Wind of about 50 L/min. is introduced into inner tub 14 by blowing fan 7. At this time, clothing 13 weighs about 4 kg based on dry weight, and weighs about 5.6 kg in a state where water is contained. The electrolytic mist can be generated at a level of about 12 ml/min by piezoelectric element 4, and variation in volume of generated mist is caused to some extent depending on the water level. The mist supply step continues until the water level sensor senses the predetermined water level, and when it is sensed, piezoelectric element 4 and blowing fan 7 stop. For example, by sensing timing when the water level reaches recessed part 5 from the bottom surface of electrolytic cell 1, a volume of the electrolytic ion water to be subsequently discharged can be reduced.

The mist will be attached to entire clothing 13 by drive motor 15 for about 10 minutes while clothing 13 is tumbled inside inner tub 14. An Ag concentration of the electrolytic ion water is high to some extent, and also, the water contained in the clothing allows the electrolytic ion water after the attachment to the clothing to wet and spread to a portion to which the mist is not attached, and thus, tumbling for about 10 minutes puts the entire clothing of 4 kg into a state where the electrolytic ion water is almost uniformly attached thereto.

The mist generated by the piezoelectric element is made of very small particles having an average particle diameter of 10 µm, which is in a white smoke-like state. A user can thus observe the mist processing step through the door of the washing and drying machine. Moreover, since the mist intrudes between inner tub 14 and outer tub 12 to be attached to some extent, periodically utilizing the mist processing function effectively brings about disinfecting and antibacterial effects on inner tub 14 and outer tub 12, and utilizing the electrolytic mist generating device allows the washing tub to be continuously maintained in a state free of mold.

Moreover, as an improved additional value of the heat pump type washing machine, even in a case where dehumidification for a room where the washing machine is installed is performed, since the mist processing function maintains the disinfecting and mold-free state inside the washing machine, nasty smell is restrained from being caused even when the washing machine inside is utilized as a blower circuit.

Thereafter, as a drainage step, the electrolytic ion water remaining in recessed part 5 is discharged from drain pipe 61 through water supply and drain port 9 and switching valve 60 to end a series of operation.

Since the mist supply step to clothing 13 is performed after spin drying operation of the washing and drying machine, the water supply step and the electrolysis step are performed prior to the mist supply step.

Evaluations of the antibacterial effect were made for the clothing subjected to the mist processing. For the evaluations, a quantitative testing method based on JIS L1902 was referred to. The evaluations were made by sewing desized testing fabrics on clothing of 4 kg with thread in ten positions. As a result, in all the fabrics, a bacteriostatic activity value of 2 or more could be obtained.

### (Embodiment 2)

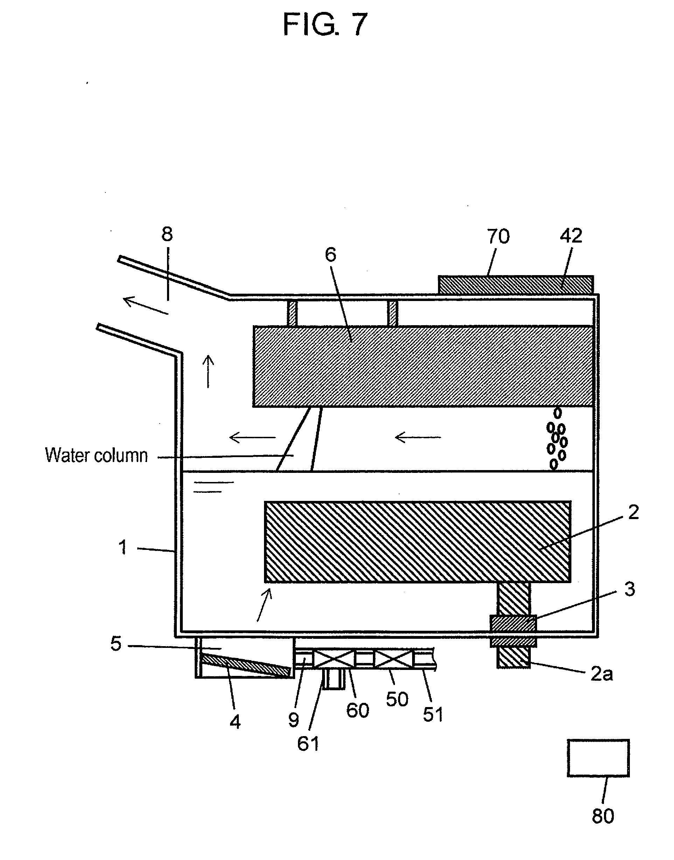

In the present embodiment, an electrolytic mist generating device resembles that of the first embodiment, and thus, a detailed description of the entire device is omitted. Fig. 7 is a schematic configuration view of the electrolytic mist generating device in a second embodiment of the present invention.

In discharging device 70, a blowing fan is not provided, but intake port 42 is provided. A configuration is adapted such that the inside of electrolytic cell 1 is put into a negative pressure state to take in air from intake port 42 and that the generated mist is discharged outside of electrolytic cell 1. When a description is given to the washing machine shown in Fig. 4, by operating blower 19, a negative pressure force acts on the mist outlet 8 side of electrolytic mist generating device 40 so that the mist generated inside electrolytic cell 1 is directed to the inner tub 14 side.

### (Embodiment 3)

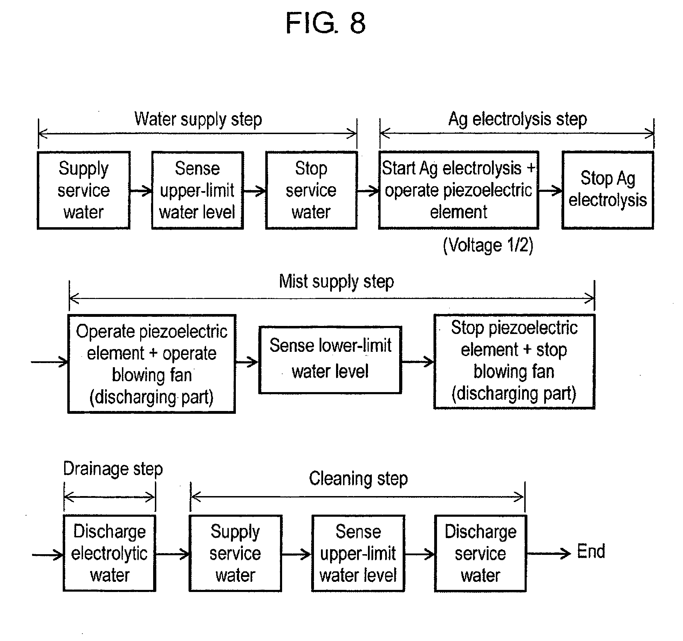

Also, in the present embodiment, an electrolytic mist generating device resembles that of the first embodiment, and thus, a detailed description of the entire device is omitted. As a different point from the first embodiment, operation of the electrolytic mist generating device is described. Fig. 8 is a system flowchart showing the operation of the electrolytic mist generating device.

Here, after the drainage step, a cleaning step is added. Specifically, after the drainage step, service water is supplied from water supply and drain port 9 up to a predetermined water level. Once the predetermined water level is sensed by the water level sensor, the switching valve is turned to a drainage direction to discharge the stored water at a time. This allows electrode part 2 surfaces used in the electrolysis, an electrolytic cell 1 inner surface or a piezoelectric element 4 surface to be cleaned in each use. Accordingly, even when a use interval of the electrolytic mist generating device becomes long, deposit accumulation can be restrained in long-term use because the inside of the electrolytic mist generating device has been cleaned. Even if the deposit accumulation occurs, the cleaning step allows the deposits to be discharged outside.

### (Embodiment 4)

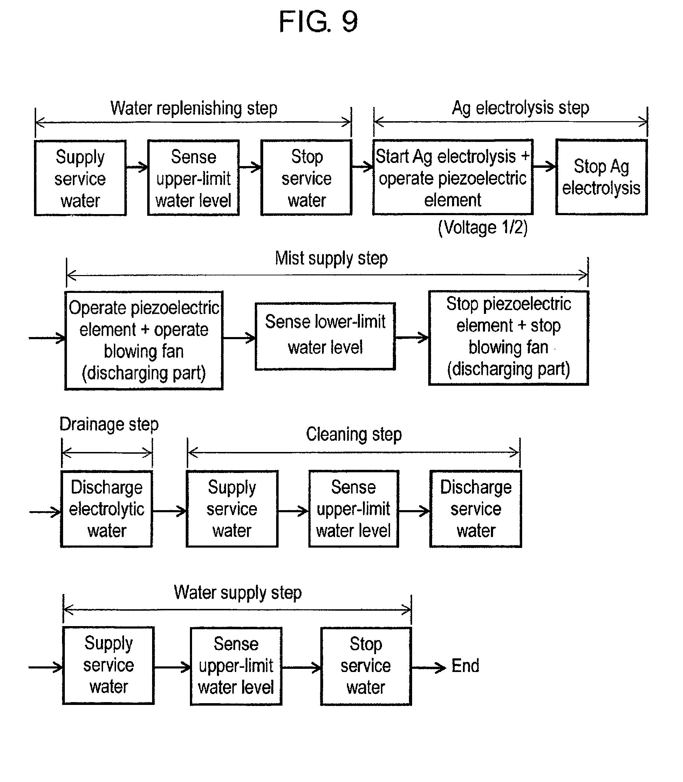

Also, in the present embodiment, an electrolytic mist generating device resembles that of the first embodiment, and thus, a detailed description of the entire device is omitted. As a different point from the first embodiment, operation of the electrolytic mist generating device is described. Fig. 9 is a system flowchart showing the operation of the electrolytic mist generating device.

Here, after the series of electrolytic mist generating operation ends, electrolytic cell 1 is put into a state where water is stored to prepare for the next use. Accordingly, as a first step, the processing starts with a water replenishing step of replenishing shortage in volume of water with respect to the predetermined water level. Thereafter, the operation proceeds as in the second embodiment, and after the cleaning step, a water supply step is added. This will allow the electrode part 2 surfaces used in the electrolysis, the electrolytic cell 1 inner surface or the surface of piezoelectric element 4 to be cleaned in each use.

Finally, since electrolytic cell 1 is put into the state where the water is stored to prepare for the next use, the inside of the electrolytic mist generating device is not dry even if the use interval of the electrolytic mist generating device becomes long. Thereby, the device can be maintained in a state where solid materials hardly occur even in a long-term use. Even if deposits occur, they can be discharged outside in the cleaning step.

While in the present embodiment, the water supply step is performed after the cleaning step, in a system flow in which the water supply step is added after the drainage step, similarly, the inside of the electrolytic mist generating device is not dry even if the use interval of the electrolytic mist generating device becomes long, and thus, the device can be maintained in a state where solid materials hardly occur even in a long-term use.

While in the embodiments of the present invention, the piezoelectric element of 1.6 MHz is used, one that can be used in the present invention is not limited to this. 1.6 MHz is common in a piezoelectric element for use in a humidifier or the like, and produces a mist particle diameter of an average of about 4 µm. In addition to this, 2.4 MHz or 1 MHz is usable. However, with 2.4 MHz, a range of the water level enabling the mist to be generated is smaller as compared with 1.6 MHz. Moreover, with 1 MHz, a water level required for protecting the piezoelectric element from being destroyed due to idling becomes higher, and thus, the depth of the recessed part needs to larger.

While in the present embodiments of the present invention, the piezoelectric element is arranged by providing the recessed part having the depth of 15 mm from the bottom surface of the electrolytic mist generating cell, the arrangement that can be used in the present invention is not limited to this. A point to notice in the mist generation from the piezoelectric element is that an upper surface of the piezoelectric element should not be in a boil-dry state in operation. Accordingly, a certain lower-surface water level enabling the usage is required, and as a result, the electrolytic water remains each time the electrolytic mist generating device is used. It is desirable to discard the remaining electrolytic water instead of leaving it as it is. Thus, providing the recessed part in the piezoelectric element part of the electrolytic mist generating cell can reduce a discarded volume of electrolytic water as much as possible. Moreover, by optimizing the depth of the recessed part in a range of 10 mm or more and 30 mm or less, the mist generation is enabled down to a border line between the bottom surface of the electrolytic mist generating cell and the recessed part.

While in the embodiments of the present invention, the piezoelectric element holds the inclination of 10 degrees with respect to the electrolytic cell bottom surface, the inclination that can be used in the present invention is not limited to this. It is considered to be desirable that the piezoelectric element has an inclination of at least 5 degrees or more inclination for effectively detaching the mist from the water column generated from the piezoelectric element. Moreover, although it is preferable that the inclination is larger to some extent for directing the circulating water in a electrode part direction adjacent to the piezoelectric element, too large an inclination reduces an effective water-level range enabling the mist generation, and thus, it is considered to be desirable that the inclination is set up to about 30 degrees.

While in the embodiments of the present invention, in the mist supply step, the predetermined lower surface water level is set to the border with respect to the recessed part provided in the electrolytic cell bottom surface, it is not limited to this. After the mist generation, however, discarding the remaining electrolytic water instead of leaving the same until the next use advantageously causes no trouble. Accordingly, setting the water level to the bottom surface of the electrolytic cell or lower is desirable because of less wasteful water with the metal ion eluted.

While in the embodiments of the present invention, the substantially semi-cylindrical rectifier made of glass is used, one that can be used in the present invention is not limited to this. Any material that does not so largely attenuate energy that the water column possesses may be employed. A resin material is not preferable because it considerably absorbs and attenuates the energy that the water column possesses. In addition to glass, aluminum, stainless steel, ceramics and the like can be used.

While in the embodiments of the present invention, as the voltage that does not permit the piezoelectric element to generate the mist, 1/2 of the rated voltage for use in mist generation is employed, the voltage is not limited to this. Once the voltage exceeds 1/2, a volume of generated mist gradually becomes larger, which, in some cases, gives rise to a need for regulation to inhibit the generated mist from flowing outside. Less than 3/1 does not produce the water column from the water surface, resulting in a remarkable deterioration in a circulating state of the water. Accordingly, with the voltage that does not permit the mist to be generated, a range of 1/3 or more and 1/2 or less of the rated voltage is preferable.

While in the embodiments of the present invention, the electrolysis step is controlled by the constant current circuit, the step is not limited to this. As in Japan, in a case where the service water is soft water and characteristics thereof is constant to some extent, the constant current circuit can make effects by the Ag processing more uniform, while when the hardness of water is higher as is often a case in other countries, electrolysis at a constant voltage can make the effects by the Ag processing more uniform.

While in the embodiments of the present invention, the electrolytic mist generating device is disposed in the front-surface-side upper portion of the oblique washing tub, the position is not limited to this. However, since disposing the electrolytic mist generating device in the front-surface-side upper portion allows the user to observe contact of the mist having an average particle diameter of 10 µm or less with washed clothing inside the washing tub, the user can visually check the clothing processing step with the mist.

While in the embodiments of the present invention, as the electrolysis step, the Ag ion water of 50 ppm is used, the concentration is not limited to this. Ag of 1 mg needs to be carried for clothing of 1 kg for stably obtaining the antibacterial effect in the clothing, and thus, Ag of 4 mg or more is needed for standard clothing of 4 kg. Moreover, a volume of mist that can be generated by the piezoelectric element is about 15 g/min. or less. Although dilute Ag ion water is preferable for evenly carrying Ag on the clothing, processing time becomes longer. If the Ag ion water is concentrated excessively, it turns brownish from cloudy, which causes a concern of the harmful effect on the clothing. Accordingly, in light of the volume of the generated mist, the processing time and the harmful effect on the clothing, as a AG ion concentration, the usage at a level of 20 ppm or more and 200 ppm or less in 250 ml or less and 25 ml or more for the standard clothing of 4 kg is considered to be preferable.

While in the embodiments of the present invention, the mist supply step is performed while tumbling is performed after the final spin drying step, the step is not limited to this. However, after the electrolytic ion aqueous solution is attached to the washed clothing, the electrolytic ion aqueous solution further wets and spreads by utilizing contained water in the clothing, and even if unevenness in the mist attachment occurs, it could be corrected. Thus, reduction in the processing time can be achieved by utilizing high-concentration Ag ion water.

### INDUSTRIAL APPLICABILITY

As described above, the electrolytic mist generating device of the present invention, which is a device capable of stably and continuously producing high-concentration metal ion water, can be applied to a wide range of applications such as an air washer, an air conditioner or water-related equipment requiring disinfection.

## Claims

1. An electrolytic mist generating device comprising:
an electrolytic cell;
electrode parts where a pair of positive pole and negative pole are arranged in parallel and opposed to one another in the electrolytic cell;
a piezoelectric element that generates mist of electrolytic water generated inside the electrolytic cell;
a water supply device that supplies water into the electrolytic cell;
a discharging device that discharges the mist from the electrolytic cell; and
a control circuit that controls steps,
wherein in an electrolysis step, a voltage that does not permit the mist to be generated is applied to the piezoelectric element to generate a water column.

2. The electrolytic mist generating device according to claim 1,
wherein the control circuit executes a drainage step of discharging electrolytic water in the electrolytic cell.

3. The electrolytic mist generating device according to claim 2,
wherein the control circuit executes a water supply step of supplying water into the electrolytic cell after the drainage step.

4. The electrolytic mist generating device according to claim 2,
wherein the control circuit executes a cleaning step of cleaning an inside of the electrolytic cell after the drainage step.

5. The electrolytic mist generating device according to claim 4,
wherein the control circuit executes a water supply step of supplying water into the electrolytic cell after the cleaning step.

6. The electrolytic mist generating device according to claim 1,
wherein the voltage that does not permit the mist to be generated and is applied to the piezoelectric element is 1/3 or more and 1/2 or less of a rated voltage permitting the mist to be generated.

7. The electrolytic mist generating device according to claim 1,
wherein the water column generated from the piezoelectric element is formed by a water flow passing though the electrode parts.

8. The electrolytic mist generating device according to claim 1,
wherein the electrolytic cell has a recessed part having a predetermined depth in a bottom portion thereof, and the piezoelectric element is arranged so as to be inclined in the recessed part.

9. The electrolytic mist generating device according to claim 1,
wherein the electrolysis step is controlled by a constant current circuit.

10. A washing machine comprising:
the electrolytic mist generating device according to claim 1;
a washing tub that contains laundry;
an outer tub in which the washing tub is internally mounted rotatably; and
a water supply device that supplies washing water to the outer tub,
wherein mist of electrolytic water generated by the electrolytic mist generating device is supplied to the washing tub.

11. The washing machine according to claim 10, wherein an inner surface of an introduction part from the electrolytic mist generating device to the washing tub is subjected to water repellent treatment.

12. The washing machine according to claim 10, wherein the electrolytic mist generating device supplies mist of Ag ion electrolytic water of 20 ppm or more and 200 ppm or less.

13. The washing machine according to claim 10, wherein the supply of the mist to the washing tub is performed while laundry is tumbled after a final spin drying step.

## Patentansprüche

1. Vorrichtung zur Erzeugung eines elektrolytischen Nebels, die Folgendes umfasst:
eine elektrolytische Zelle,
Elektrodenteile, von denen ein Paar aus einem positiven Pol und einem negativen Pol besteht, die parallel und einander gegenüberliegend in der elektrolytischen Zelle angeordnet sind,
ein piezoelektrisches Element, das innerhalb der elektrolytischen Zelle einen Nebel aus elektrolytischem Wasser erzeugt,
eine Wassereinspeiseeinrichtung, die Wasser in die elektrolytische Zelle einspeist,
eine Abgabeeinrichtung, die den Nebel aus der elektrolytischen Zelle abgibt, und
einen Steuerungsschaltkreis, der die Schritte steuert,
wobei eine elektrische Spannung, die eine Erzeugung des Nebels nicht zulässt, in einem Elektrolyseschritt an das piezoelektrische Element angelegt wird, um eine Wassersäule zu erzeugen.

2. Vorrichtung zur Erzeugung eines elektrolytischen Nebels nach Anspruch 1, wobei der Steuerungsschaltkreis einen Abflußschritt ausführt, wonach elektrolytisches Wasser in der elektrolytischen Zelle abgegeben wird.

3. Vorrichtung zur Erzeugung eines elektrolytischen Nebels nach Anspruch 2, wobei der Steuerungsschaltkreis einen Wassereinspeiseschritt ausführt, wonach nach dem Abflußschritt Wasser in die elektrolytische Zelle eingespeist wird.

4. Vorrichtung zur Erzeugung eines elektrolytischen Nebels nach Anspruch 2, wobei der Steuerungsschaltkreis einen Reinigungsschritt ausführt, wonach nach dem Abflußschritt das Innere der elektrolytischen Zelle gereinigt wird.

5. Vorrichtung zur Erzeugung eines elektrolytischen Nebels nach Anspruch 4, wobei der Steuerungsschaltkreis einen Wassereinspeiseschritt ausführt, wonach nach dem Reinigungsschritt Wasser in die elektrolytische Zelle eingespeist wird.

6. Vorrichtung zur Erzeugung eines elektrolytischen Nebels nach Anspruch 1, wobei die Spannung, die eine Erzeugung des Nebels nicht zulässt und an das piezoelektrische Element angelegt wird, zwischen einem Drittel und einer Hälfte einer Nennspannung beträgt, die eine Erzeugung des Nebels zulässt.

7. Vorrichtung zur Erzeugung eines elektrolytischen Nebels nach Anspruch 1, wobei die Wassersäule, die vom piezoelektrischen Element erzeugt wird, **dadurch** gebildet wird, dass ein Wasserstrom die Elektrodenteile durchfließt.

8. Vorrichtung zur Erzeugung eines elektrolytischen Nebels nach Anspruch 1, wobei die elektrolytische Zelle eine Vertiefung aufweist, deren unterer Abschnitt eine vorbestimmte Tiefe aufweist, und das piezoelektrische Element so angeordnet ist, dass es geneigt in der Vertiefung liegt.

9. Vorrichtung zur Erzeugung eines elektrolytischen Nebels nach Anspruch 1, wobei der Elektrolyseschritt durch einen Konstantstromschaltkreis gesteuert wird.

10. Waschmaschine, die Folgendes umfasst:
die Vorrichtung zur Erzeugung eines elektrolytischen Nebels nach Anspruch 1,
eine Wäsche beinhaltende Waschwanne,
eine Außenwanne, in der die Waschwanne innen drehbar gelagert ist, und
eine Wassereinspeiseeinrichtung, die Waschwasser in die Außenwanne einspeist,
wobei ein Nebel aus elektrolytischem Wasser, der von der Vorrichtung zur Erzeugung eines elektrolytischen Nebels erzeugt wird, in die Waschwanne eingespeist wird.

11. Waschmaschine nach Anspruch 10, wobei eine Innenfläche eines Einführteils, der von der Vorrichtung zur Erzeugung eines elektrolytischen Nebels bis zur Waschwanne reicht, einer Wasserabweisungsbehandlung unterzogen wird.

12. Waschmaschine nach Anspruch 10, wobei die Vorrichtung zur Erzeugung eines elektrolytischen Nebels einen Nebel aus elektrolytischem Wasser einspeist, das Ag-Ionen in einer Konzentration zwischen 20 ppm und 200 ppm aufweist.

13. Waschmaschine nach Anspruch 10, wobei der Nebel in die Waschwanne eingespeist wird, während die Wäsche nach einem letzten Trockenschleuderschritt im Tumbler getrocknet wird.

## Revendications

1. Dispositif générateur de brouillard électrolytique comprenant :
une cellule électrolytique;
des parties d'électrode où une paire de pôle positif et de pôle négatif sont agencés parallèlement et l'un en face de l'autre dans la cellule électrolytique ;
un élément piézoélectrique qui génère un brouillard d'eau électrolytique généré à l'intérieur de la cellule électrolytique ;
un dispositif d'alimentation en eau qui délivre de l'eau dans la cellule électrolytique ;
un dispositif de décharge qui décharge le brouillard de la cellule électrolytique ; et
un circuit de commande qui commande les étapes,
dans lequel, à une étape d'électrolyse, une tension qui ne permet pas la génération du brouillard est appliquée à l'élément piézoélectrique pour générer une colonne d'eau.

2. Dispositif générateur de brouillard électrolytique selon la revendication 1, dans lequel le circuit de commande exécute une étape de drainage consistant à décharger de l'eau électrolytique dans la cellule électrolytique.

3. Dispositif générateur de brouillard électrolytique selon la revendication 2, dans lequel le circuit de commande exécute une étape d'alimentation en eau consistant à délivrer de l'eau dans la cellule électrolytique après l'étape de drainage.

4. Dispositif générateur de brouillard électrolytique selon la revendication 2, dans lequel le circuit de commande exécute une étape de nettoyage consistant à nettoyer l'intérieur de la cellule électrolytique après l'étape de drainage.

5. Dispositif générateur de brouillard électrolytique selon la revendication 4, dans lequel le circuit de commande exécute une étape d'alimentation en eau consistant à délivrer de l'eau dans la cellule électrolytique après l'étape de nettoyage.

6. Dispositif générateur de brouillard électrolytique selon la revendication 1, dans lequel la tension qui ne permet pas la génération du brouillard et qui est appliquée à l'élément piézoélectrique est égale à 1/3 ou plus et 1/2 ou moins d'une tension nominale permettant la génération du brouillard.

7. Dispositif générateur de brouillard électrolytique selon la revendication 1, dans lequel la colonne d'eau générée par l'élément piézoélectrique est formée par une circulation d'eau passant à travers les parties d'électrode.

8. Dispositif générateur de brouillard électrolytique selon la revendication 1, dans lequel la cellule électrolytique comporte une partie évidée ayant une profondeur prédéterminée dans une partie inférieure de celle-ci, et l'élément piézoélectrique est agencé de manière à être incliné dans la partie évidée.

9. Dispositif générateur de brouillard électrolytique selon la revendication 1, dans lequel l'étape d'électrolyse est commandée par un circuit de courant constant.

10. Machine à laver comprenant :
le dispositif générateur de brouillard électrolytique selon la revendication 1 ;
une cuve de lavage qui contient le linge ;
une cuve externe dans laquelle la cuve de lavage est montée intérieurement en rotation ; et
un dispositif d'alimentation en eau qui délivre de l'eau de lavage à la cuve externe,
dans laquelle le brouillard d'eau électrolytique généré par le dispositif générateur de brouillard électrolytique est délivré à la cuve de lavage.

11. Machine à laver selon la revendication 10, dans laquelle une surface interne d'une partie d'introduction du dispositif générateur de brouillard électrolytique à la cuve de lavage est soumise à un traitement hydrofuge.

12. Machine à laver selon la revendication 10, dans laquelle le dispositif générateur de brouillard électrolytique délivre un brouillard d'eau électrolytique contenant des ions d'Ag de 20 ppm ou plus et de 200 ppm ou moins.

13. Machine à laver selon la revendication 10, dans laquelle la fourniture du brouillard à la cuve de lavage est effectuée alors que le linge est mélangé dans le tambour après une étape d'essorage finale.
